(19) Europäisches Patentamt
European Patent Office
Office européen des brevets

(11) Numéro de publication : **0 155 870**
**B1**

# (12) FASCICULE DE BREVET EUROPÉEN

(45) Date de publication du fascicule du brevet : **26.04.89**

(51) Int. Cl.[4] : **C 07 D205/04**, C 07 D403/04

(21) Numéro de dépôt : **85400313.4**

(22) Date de dépôt : **21.02.85**

(54) **3-aminoazétidine, ses sels, procédé pour leur préparation et intermédiaires de synthèse.**

(30) Priorité : **27.02.84 FR 8402951**

(43) Date de publication de la demande :
**25.09.85 Bulletin 85/39**

(45) Mention de la délivrance du brevet :
**26.04.89 Bulletin 89/17**

(84) Etats contractants désignés :
**AT BE CH DE FR GB IT LI LU NL SE**

(56) Documents cités :
**FR-A- 2 150 816**
**CHEMICAL ABSTRACTS, vol. 83, no. 1, 7 juillet 1975, page 811,no. 9760u, Columbus, Ohio, US; & JP - A - 74 109 369 (TEIKOKU HORMONE MFG. CO., LTD.) 17-10-1974**

(73) Titulaire : **SANOFI**
**40, Avenue George V**
**F-75008 Paris (FR)**
**BE CH DE FR GB LI LU NL SE AT**
**MIDY S.p.A., Société Anonyme dite:**
**Via Piranesi 38**
**I-20137 Milano (IT)**
**IT**

(72) Inventeur : **Nisato, Dino**
**4 Impasse de l'Olivette**
**F-34680 St. Georges d'Orques (FR)**
Inventeur : **Frigerio, Marco**
**Via Carlo Poma 28**
**Mantova (IT)**

(74) Mandataire : **Gillard, Marie-Louise et al**
**Cabinet Beau de Loménie 55, Rue d'Amsterdam**
**F-75008 Paris (FR)**

**EP 0 155 870 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la 3-aminoazétidine de formule I

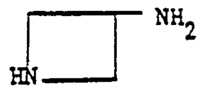

ses sels, un procédé pour leur préparation et les intermédiaires utilisables dans la synthèse du produit.

La 3-aminoazétidine n'a jamais été décrite en littérature. En effet, le noyau azétidinique est particulièrement sensible aux conditions hydrolytiques qui provoquent son ouverture en présence d'eau ou d'un radical nucléophile (C. Mannich, G. Baumgarter, Chem. Ber. 1937, 70, 210-213). Il est aussi possible que l'ouverture du noyau donne lieu à une réaction de polymérisation (C.F. Gibbs, C.S. Harvel J., Am. Chem. Soc. 1935, 57, 1137-1139).

Ainsi, on peut préparer, selon des méthodes connues, une 3-aminoazétidine qui est substituée sur le groupe amino primaire par des radicaux différents, mais la littérature n'enseigne pas comment préparer une azétidine ayant un groupe amino libre en position 3.

Dans la demande de brevet japonais, publiée sous le numéro 74-109 369, il est décrit et revendiqué un procédé de préparation de nouvelles 3-amino azétidine répondant à la formule :

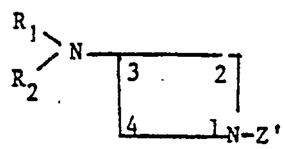

(I)

Il est spécifiquement indiqué que $R_1$, $R_2$ et Z' ne peuvent être, en même temps, l'hydrogène.

De plus, le procédé tel que décrit dans ladite demande, avec ses variantes, ne permet pas la préparation de la 3-aminozétidine libre car, comme indiqué ci-dessus, il provoquerait l'ouverture du cycle azétidinique.

On notera également qu'une seule azétidine portant un groupe amino libre en position 3 est décrite dans ce document : la 3-amino-3-éthylazétidine, qui ne peut être utilisée comme intermédiaire pour la préparation de la 3-aminoazétidine.

On a maintenant trouvé que la 3-aminoazétidine peut être préparée à partir d'une 3-sulfonyloxyazétidine dont l'azote est protégé. Par substitution du groupe sulfonyloxy avec un groupe phtalimido, on obtient des intermédiaires nouveaux qui, par deux réactions successives d'hydrolyse et d'hydrogénation catalytique, donnent la 3-aminoazétidine, sous forme d'un de ses sels.

Ainsi, selon un de ses aspects, la présente invention a pour objet la 3-aminoazétidine de formule I ci-dessus et ses sels d'addition avec des acides inorganiques ou organiques, tels que le chlorhydrate, le bromhydrate, le sulfate, l'acétate, le maléate, le fumarate, l'oxalate, le picrate, le méthanesulfonate et similaires.

Ces produits sont utiles dans la préparation de composés correspondants 1-substitués à action psychotrope. Plus particulièrement, par protection de l'azote cyclique sous forme de dérivé benzhydryle, on prépare le dérivé 3-acétylamino qui, après élimination du groupe benzhydryle et réaction successive avec la 2,6-dichloropyridine, donne un composé qui, par hydrolyse acide, fournit la 3-amino-1-(6-chloropyrid-2-yl)-azétidine douée d'une activité anorexigène à la dose de 15 mg/kg par voie orale dans le test de la prise de nourriture chez le rat.

Selon un autre de ses aspects, la présente invention a pour objet un procédé pour la préparation de la 3-aminoazétidine et de ses sels, caractérisé en ce que l'on traite une 3-sulfonyloxyazétidine protégée en position 1 de formule II

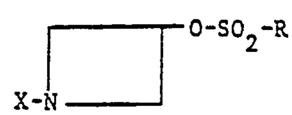

(II)

dans laquelle R représente un groupe alkyle contenant de 1 à 4 atomes de carbone, un groupe phényle ou un groupe tolyle et X représente un groupe protecteur, avec un phtalimide alcalin, on soumet la 3-phtalimidoazétidine protégée en position 1 à deux réactions successives d'hydrolyse avec une solution aqueuse d'hydrazine à 60-90 % et d'hydrogénation catalytique ou, lorsque le groupe protecteur est le groupe trityle, d'hydrolyse dans des conditions acides douces et l'on transforme le produit ainsi obtenu en ses sels d'addition avec des acides inorganiques ou organiques.

Les groupes protecteurs comprennent le benzyle, le benzhydryle ou le trityle éventuellement substitué par le groupe méthoxy et le 2,2,2-trichloroéthyle.

Le procédé de la présente invention est représenté par le schéma suivant :

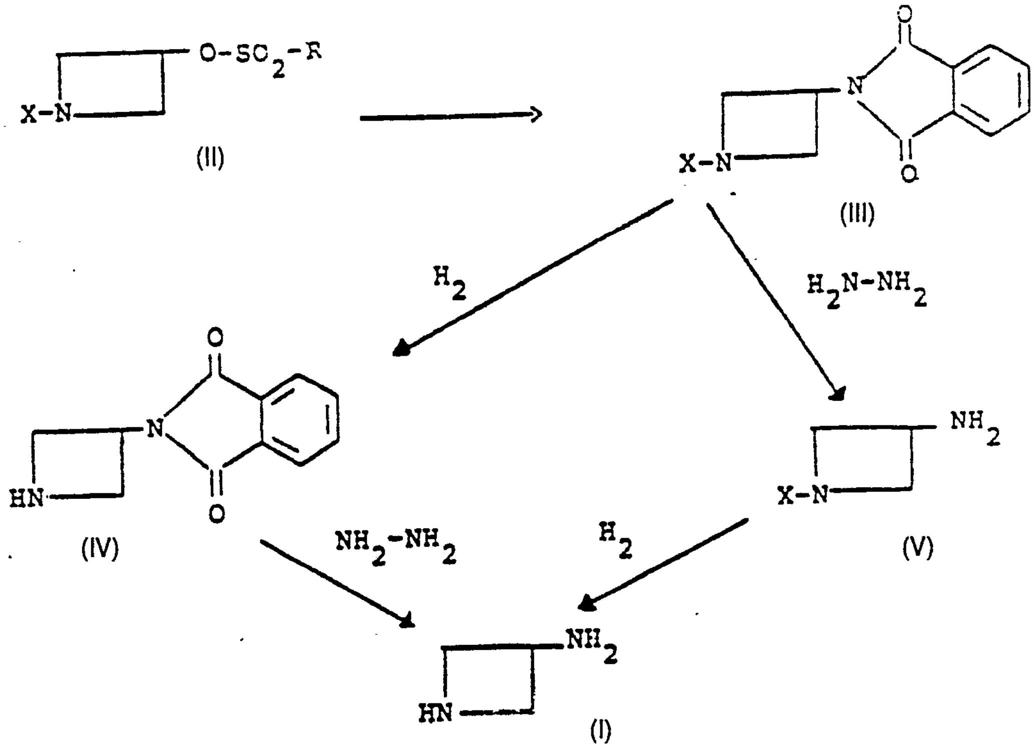


(II) (III) (IV) (V) (I)

où X et R sont tels que définis ci-dessus.

La réaction entre la 3-sulfonyloxyazétidine 1-substituée II et le phtalimide alcalin, de préférence le phtalimide potassique, est effectuée dans un solvant organique, tel que le toluène, l'hexane et similaires en présence d'un catalyseur de transfert de phase, tel qu'un sel d'ammonium ou de phosphonium, par exemple le bromure de tétrabutylammonium ou le bromure d'hexadécyltributylphosphonium à une température de 100 à 140 °C.

La 3-phtalimidoazétidine 1-substituée ainsi obtenue peut être traitée de façon à débloquer le groupe protecteur. Le déblocage est généralement effectué par hydrogénation catalytique en utilisant de préférence, comme catalyseur, le palladium hydroxyde sur charbon en présence d'acide chlorhydrique.

La 3-phtalimidoazétidine IV ainsi obtenue est hydrolysée par action de l'hydrazine. L'hydrolyse est conduite dans un solvant organique, tel que le méthanol, en utilisant l'hydrate d'hydrazine.

Selon une variante du procédé de la présente invention, la 3-phtalimidoazétidine 1-substituée de formule III est d'abord soumise à la réaction d'hydrolyse avec l'hydrazine dans les conditions décrites ci-dessus.

La 3-aminoazétidine 1-substituée V ainsi obtenue est ensuite débloquée comme décrit ci-dessus pour le composé III et la 3-aminoazétidine est isolée sous forme d'un de ses sels.

Lorsque le groupe protecteur X présent dans les composés III et V est un groupe trityle éventuellement substitué, le déblocage peut être effectué dans des conditions douces d'hydrolyse acide, par exemple par action d'une solution aqueuse d'acide formique à 50 % à une température de 20 à 50 °C.

La 3-aminoazétidine peut être isolée sous forme de base libre très volatile, à partir des sels correspondants, et caractérisée chimiquement en la transformant dans son dérivé 1,N-dibenzoylé.

Selon un autre de ses aspects, la présente invention a pour objet des intermédiaires utiles pour la synthèse de la 3-aminoazétidine et de ses dérivés sur l'amine primaire, caractérisés par la formule VI

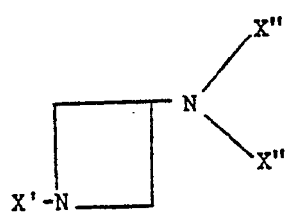

(VI)

dans laquelle X′ représente l'hydrogène ou un groupe protecteur choisi parmi les groupes benzyle, benzhydryle ou trityle éventuellement substitués par le groupe méthoxy, les deux X″ représentent l'hydrogène ou, avec l'atome d'azote auquel ils sont attachés, un groupe phtalimido X′ et les deux X″

n'étant pas en même temps l'hydrogène, et leurs sels d'addition avec des acides inorganiques ou organiques.

Les exemples suivants illustrent l'invention sans toutefois la limiter. Les spectres RMN ont été obtenus à l'aide d'un appareil WP-80 CW Brucker à 80 MHz à une concentration de 5 % p/v.

### Exemple 1

On chauffe au reflux pendant 5 h un mélange de 0,362 mol de 1-benzhydryl-3-mésyloxyazétidine (J. Org. Chem., 1972, 37, 3953-3955), 0,44 mol de phtalimide potassique et 0,045 mol de bromure d'hexadécyltributylphosphonium dans 500 ml de toluène anhydre, puis on refroidit à 20 °C. On filtre le précipité, on le lave avec 400 ml d'acétate d'éthyle et on l'élimine. On lave la solution organique avec de l'eau, on la sèche sur du sulfate de sodium anhydre, on la filtre sur charbon et on la concentre sous pression réduite. On cristallise le résidu huileux avec 600 ml d'éther diisopropylique. On obtient ainsi 80 g (rendement : 67,4 % de la théorie) de 1-benzhydryl-3-phtalimidoazétidine ; p.f. 118-120 °C. Des eaux mères on obtient 10 g supplémentaires de produit.

IR : $\nu$ max (KBr) : 1750, 1710 (b), 1 390 $cm^{-1}$.

1H-RMN ($CDCl_3$) : δTMS (ppm) : 3,64 (4H, m→d), 4,63 (1H, s), 4,83 (1H, m→t), 7,0-7,8 (14H, m).

De la même façon, on obtient le même produit, avec un rendement de 20 %, en utilisant le bromure de tétrabutylammonium comme catalyseur de transfert de phase.

### Exemple 2

On chauffe au reflux pendant 15 min 0,244 mol de 1-benzhydryl-3-phtalimidoazétidine dans 1 000 ml de méthanol anhydre, puis on éloigne le bain chauffant et on ajoute 0,339 mol d'hydrate d'hydrazine à 85 %. On chauffe le mélange au reflux pendant 30 min, puis un produit solide blanc commence à précipiter de la solution limpide ainsi obtenue. On chauffe encore le mélange pendant 90 min, puis on le refroidit à 10 °C. On ajoute 1 litre d'éther diéthylique, on filtre et on lave avec de l'éther. On élimine le produit solide (35 g) ainsi obtenu et l'on concentre les eaux mères sous pression réduite. On reprend le résidu avec 500 ml d'eau et on ajuste le pH de la solution à 11 avec de l'hydroxyde de sodium. On extrait avec 1 litre d'éther diéthylique, on lave la phase éthérée à l'eau, on la sèche sur du sulfate de sodium anhydre et on l'évapore sous pression réduite. On obtient la 3-amino-1-benzhydrylazétidine sous forme d'une huile qui tend à solidifier. Rendement 96,3 %. Par réaction avec de l'acide chlorhydrique dans de l'isopropanol/acétone on obtient le dichlorhydrate de 3-amino-1-benzhydrylazétidine qui, après cristallisation dans de l'isopropanol, fond à 108-110 °C.

1H-RMN ($CDCl_3$) : δTMS (ppm) : 1,65 [2H, s(b)], 2,6 (2H, m), 3,5 (3H, m), 4,25 (1H, s), 7,0-7,5 (10H, m) (base libre).

### Exemple 3

Un mélange de 0,044 mol de 3-amino-1-benzhydrylazétidine, 110 ml de méthanol anhydre, 2,8 g d'hydroxyde de palladium sur charbon (catalyseur de Pearlman, Lot Midy E 21/13 — W.M. Pearlman, Tetrahedron Letters 1967, 1663-1664) et 3,6 ml d'acide chlorhydrique concentré est hydrogéné à 35-40 °C pendant 5 h sous une pression de 4 bars. Le mélange réactionnel est ensuite filtré et concentré sous pression réduite à une température extérieure de 40 °C. Le résidu semi-solide est repris avec 110 ml d'isopropanol. Le dichlorhydrate de 3-aminoazétidine ainsi obtenu est filtré et séché.

Rendement : 4,9 g (76,7 %) ; p.f. 159-162 °C (déc).

1H-RMN ($DMSO-d_6$) δTMS (ppm) : 4,1 (5H, m), 9,2 [5H, s(b)]

### Exemple 4

On opérant comme décrit dans l'exemple 3, par hydrogénation de la 1-benzhydryl-3-phtalimidoazétidine on obtient le chlorhydrate de 3-phtalimidoazétidine.

### Exemple 5

A un mélange de 20 g d'hydroxyde de potassium et de glace, refroidi à —5 °C, on ajoute, par portions, 14 g de 3-aminoazétidine préparée selon l'exemple 3. On décante l'huile qui se sépare et on la conserve sur des pastilles d'hydroxyde de potassium. Par distillation sous atmosphère d'argon à 1 bar, on obtient 1 g de 3-aminoazétidine base ; point d'ébullition 135-142 °C.

Le produit ainsi obtenu est dissous dans du chlorure de méthylène et la solution résultante est traitée avec deux équivalents de chlorure de benzoyle dans du chlorure de méthylène. On agite la solution à la température ambiante pendant 8 h, on reprend le résidu avec de l'éther diéthylique, on lave avec une solution aqueuse saturée de carbonate de sodium, on la sèche sur du sulfate de sodium anhydre et l'on évapore le solvant. Par cristallisation du résidu dans de l'acétate d'éthyle, on obtient la 1-benzoyl-3-benzoylaminoazétidine ; p.f. 120-122 °C.

Analyse pour $C_{17}H_{16}N_2O_2$ :
Calculé : C 72,84 H 5,75 N 9,99 % ;
Trouvé : C 72,96 H 5,87 N 9,78 %.
Les spectres IR et RMN confirment la structure du produit.

**Revendications** (pour les Etats contractants : BE, CH, DE, FR, GB, IT, LI, LU, NL, SE)

1. La 3-aminoazétidine et ses sels d'addition avec des acides inorganiques ou organiques.

2. Le dichlorhydrate de 3-aminoazétidine.

3. Procédé pour la préparation de la 3-aminoazétidine et de ses sels, caractérisé en ce que l'on traite une 3-sulfonyloxyazétidine protégée en position 1 de formule II

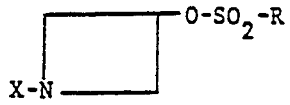

(II)

dans laquelle R représente un groupe alkyle contenant de 1 à 4 atomes de carbone, un groupe phényle ou un groupe tolyle et X représente un groupe protecteur choisi parmi les groupes benzyle, benzhydryle ou trityle éventuellement substitués par le groupe méthoxy et le 2,2,2-trichloroéthyle, avec un phtalimide alcalin, on soumet la 3-phtalimidoazétidine protégée en position 1 à deux réactions successives d'hydrolyse avec une solution aqueuse d'hydrazine à 60-90 % et d'hydrogénation catalytique ou, lorsque le groupe protecteur est trityle, d'hydrolyse dans des conditions acides douces et l'on transforme éventuellement le produit ainsi obtenu en ses sels d'addition avec des acides inorganiques ou organiques.

4. Procédé selon la revendication 3, caractérisé en ce que la 1-benzhydryl-3-mésyloxyazétidine est utilisée comme composé de départ.

5. Procédé selon la revendication 3, caractérisé en ce que l'on chauffe la 1-benzhydryl-3-mésyloxyazétidine avec un phtalimide alcalin, on hydrolyse la 1-benzhydryl-3-phtalimidoazétidine ainsi obtenue avec une solution aqueuse d'hydrazine à 60-90 %, on soumet à hydrogénation catalytique la 3-amino-1-benzhydrylazétidine ainsi obtenue et l'on transforme la 3-aminoazétidine ainsi obtenue en ses sels d'addition.

6. Procédé selon la revendication 5, caractérisé en ce que la 3-aminoazétidine est isolée sous forme de dichlorhydrate.

7. Procédé selon l'une des revendications 3 ou 5, caractérisé en ce que la réaction avec le phtalimide alcalin est effectuée en présence d'un catalyseur de transfert de phase.

8. Procédé selon la revendication 7, caractérisé en ce que le bromure de tétrabutylammonium ou le bromure d'hexadécyltributylphosphonium est utilisé comme catalyseur de transfert de phase.

9. A titre d'intermédiaires nouveaux les composés de formule VI

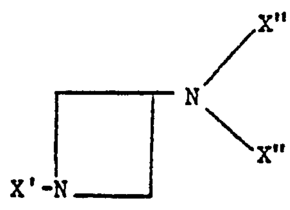

(VI)

dans laquelle X' représente l'hydrogène ou un groupe protecteur choisi parmi les groupes benzyle, benzhydryle ou trityle éventuellement substitués par le groupe méthoxy et le 2,2,2-trichloroéthyle ; les deux X" représentent l'hydrogène ou, avec l'atome d'azote auquel ils sont attachés, un groupe phtalimido X' et les deux X" n'étant pas en même temps l'hydrogène, et leurs sels d'addition avec des acides inorganiques ou organiques.

10. La 1-benzhydryl-3-phtalimidoazétidine et ses sels d'addition avec des acides inorganiques ou organiques.

11. La 3-amino-1-benzhydrylazétidine et ses sels d'addition avec des acides inorganiques ou organiques.

12. Le dichlorhydrate de 3-amino-1-benzhydrylazétidine.

13. La 3-phtalimidoazétidine et ses sels d'addition avec des acides inorganiques ou organiques.

**Revendications** (pour l'Etat contractant AT)

1. Procédé pour la préparation de la 3-aminoazétidine et de ses sels d'addition avec des acides inorganiques ou organiques, caractérisé en ce qu'il consiste à traiter une 3-sulfonyloxyazétidine protégée

en position 1 de formule II

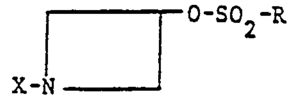

(II)

dans laquelle R représente un groupe alkyle contenant de 1 à 4 atomes de carbone, un groupe phényle ou un groupe tolyle et X représente un groupe protecteur choisi parmi les groupes benzyle, benzhydryle ou trityle éventuellement substitués par le groupe méthoxy et le 2,2,2-trichloroéthyle avec un phtalimide alcalin, à soumettre la 3-phtalimidoazétidine protégée en position 1 à deux réactions successives d'hydrolyse avec une solution aqueuse d'hydrazine à 60-90 % et d'hydrogénation catalytique ou, lorsque le groupe protecteur est trityle, d'hydrolyse dans des conditions acides douces et à transformer éventuellement le produit ainsi obtenu en ses sels d'addition avec des acides inorganiques ou organiques.

2. Procédé selon la revendication 1, caractérisé en ce que la 3-aminoazétidine est isolée sous forme de dichlorhydrate.

3. Procédé selon la revendication 1, caractérisé en ce que la 1-benzhydryl-3-mésyloxyazétidine est utilisée comme composé de départ.

4. Procédé selon la revendication 1, caractérisé en ce que l'on chauffe la 1-benzhydryl-3-mésyloxyazétidine avec un phtalimide alcalin, on hydrolyse la 1-benzhydryl-3-phtalimidoazétidine ainsi obtenue avec une solution aqueuse d'hydrazine à 60-90 %, on soumet à hydrogénation catalytique la 3-amino-1-benzhydrylazétidine ainsi obtenue et l'on transforme la 3-aminoazétidine ainsi obtenue en ses sels d'addition.

5. Procédé selon la revendication 4, caractérisé en ce que la 3-aminoazétidine est isolée sous forme de dichlorhydrate.

6. Procédé selon l'une des revendications 1 ou 4, caractérisé en ce que la réaction avec le phtalimide alcalin est effectuée en présence d'un catalyseur de transfert de phase.

7. Procédé selon la revendication 6, caractérisé en ce que le bromure de tétrabutylammonium ou le bromure d'hexadécyltributylphosphonium est utilisé comme catalyseur de transfert de phase.

8. Procédé selon l'une quelconque des revendications 1 à 7, caractérisé en ce que l'on obtient à titre d'intermédiaires des composés de formule IV :

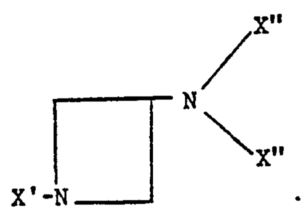

(IV)

dans laquelle X' représente l'hydrogène ou un groupe protecteur choisi parmi les groupes benzyle, benzhydryle ou trityle éventuellement substitués par le groupe méthoxy et le 2,2,2-trichloroéthyle ; les deux X" représentent l'hydrogène ou, avec l'atome d'azote auquel ils sont attachés, un groupe phtalimido X' et les deux X" n'étant pas en même temps l'hydrogène.

9. Procédé selon l'une quelconque des revendications 1 à 8, caractérisé en ce qu'il consiste à traiter la 3-sulfonyloxyazétidine protégée en position 1 par le groupe benzhydryle avec un phtalimide alcalin et à obtenir à titre de composé intermédiaire la 1-benzhydryl-3-phtalimidoazétidine.

10. Procédé selon la revendication 9, caractérisé en ce qu'il consiste à hydrolyser ladite 1-benzhydryl-3-phtalimidoazétidine et à obtenir à titre de composé intermédiaire la 3-amino-1-benzhydryl azétidine.

11. Procédé selon l'une quelconque des revendications 1 à 8, caractérisé en ce qu'il consiste à traiter la 3-sulfonyloxyazétidine protégée en position 1 avec un phtalimide alcalin, à hydrogéner la 3-phtalimidoazétidine obtenue, protégée en position 1, et à obtenir à titre de composé intermédiaire la 3-phtalimido azétidine.

**Claims** (for the Contracting States : BE, CH, DE, FR, GB, IT, LI, LU, NL, SE)

1. The 3-aminoazetidine and its addition salts with inorganic or organic acids.

2. The 3-aminoazetidine dihydrochloride.

3. Process for the preparation of 3-aminoazetidine and its salts, characterized in that it comprises the steps of :

treating a 1-protected 3-sulfonyloxyazetidine, of formula

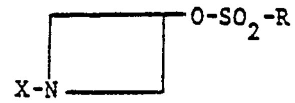

(II)

in which R represents an alkyl group containing 1 to 4 carbon atoms, a phenyl group or a tolyl group and X represents a protecting group, selected from the benzyl, benzhydryl or trityl groups optionally substituted by the methoxy group and the 2,2,2-trichloroethyl, with an alkali metal phthalimide ; submitting the 1-protected 3-phthalimidoazetidine to two subsequent reactions of hydrolysis with a 60-90 % aqueous solution of hydrazine and of catalytic hydrogenation or, when the protecting group is trityl, of hydrolysis under mild acid conditions ; and optionally converting the resulting product into its addition salts with inorganic or organic acids.

4. Process according to claim 3, characterized in that the 1-benzhydryl-3-mesyloxyazetidine is used as starting compound.

5. Process according to claim 3, characterized in that the 1-benzhydryl-3-mesyloxyazetidine is heated with an alkali metal phthalimide, the resulting 1-benzhydryl-3-phthalimidoazetidine is hydrolyzed with a 60-90 % aqueous solution of hydrazine, the resulting 3-amino-1- benzhydrylazetidine is catalytically hydrogenated and the resulting 3-aminoazetidine is converted into the addition salts thereof.

6. Process according to claim 5, characterized in that the 3-aminoazetidine is isolated in dihydrochloride form.

7. Process according to one of claims 3 or 5, characterized in that the reaction with the alkali metal phthalimide is carried out in the presence of a phase transfer catalyst.

8. Process according to claim 7, characterized in that the tetrabutylammonium bromide or the hexadecyltributylphosphonium bromide is used as phase transfer catalyst.

9. As new intermediates the compounds of formula :

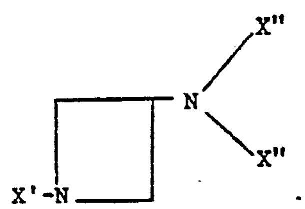

(VI)

wherein X′ represents hydrogen or a protecting group, selected from the benzyl, benzhydryl or trityl groups optionally substituted by the methoxy group and the 2,2,2-trichloroethyl, both X″ represent hydrogen or, with the nitrogen atom to which they are attached, a phthalimido group, X′ and both X″ being never hydrogen at the same time, and their addition salts with organic or inorganic acids.

10. The 1-benzhydryl-3-phthalimidoazetidine and its addition salts with organic or inorganic acids.

11. The 3-amino-1-benzhydrylazetidine and its addition salts with organic or inorganic acids.

12. The 3-amino-1-benzhydrylazetidine hydrochloride.

13. The 3-phthalimidoazetidine and its addition salts with organic or inorganic acids.

**Claims** (for the Contracting State AT)

1. Process for the preparation of 3-aminoazetidine and its addition salts, with inorganic or organic acids, characterized in that it consists in : treating a 1-protected 3-sulfonyloxyazetidine, of formula II

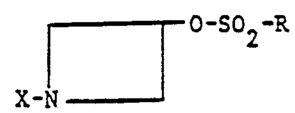

(II)

in which R represents an alkyl group containing 1 to 4 carbon atoms, a phenyl group or a tolyl group and X represents a protecting group selected from the benzyl, benzhydryl or trityl groups optionally substituted by the methoxy group and the 2,2,2-trichloroethyl, with an alkali metal phthalimide, submitting the 1-protected 3-phthalimidoazetidine to two subsequent reactions of hydrolysis with a 60-90 % aqueous solution of hydrazine and of catalytic hydrogenation or, when the protecting group is trityl, of hydrolysis under mild acid conditions and optionally converting the resulting product into its addition salts with inorganic or organic acids.

2. Process according to claim 1, characterized in that the 3-aminoazetidine is isolated in dihydrochloride form.

3. Process according to claim 1, characterized in that the 1-benzhydryl-3-mesyloxyazetidine is used as starting compound.

4. Process according to claim 1, characterized in that the 1-benzhydryl-3-mesyloxyazetidine is

heated with an alkali metal phthalimide, the resulting 1-benzhydryl-3-phthalimidoazetidine is hydrolyzed with a 60-90 % aqueous solution of hydrazine, the resulting 3-amino-1-benzhydrylazetidine is catalytically hydrogenated and the resulting 3-aminoazetidine is converted into the addition salts thereof.

5. Process according to claim 4, characterized in that the 3-aminoazetidine is isolated in dihydrochloride form.

6. Process according to one of claims 1 or 4, characterized in that the reaction with the alkali metal phthalimide is carried out in the presence of a phase transfer catalyst.

7. Process according to claim 6, characterized in that the tetrabutylammonium bromide or the hexadecyltributylphosphonium bromide is used as phase transfer catalyst.

8. Process according to any one of claims 1 to 7, characterized in that new compounds are obtained as intermediate, which compounds are of formula IV :

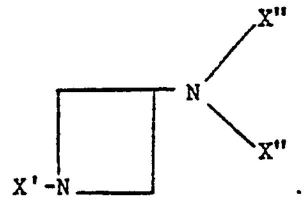

(IV)

wherein X′ represents hydrogen or a protecting group, selected from the benzyl, benzhydryl or trityl groups optionally substituted by the methoxy group and the 2,2,2-trichloroethyl, both X″ represent hydrogen or, with the nitrogen atom to which they are attached, a phthalimido group, X′ and both X″ being never hydrogen at the same time.

9. Process according to any one of claims 1 to 8, characterized in that it consists in treating 3-sulfonyloxyazetidine protected in position 1 by the benzhydryl group with an alkali metal phthalimide and in obtaining the 1-benzhydryl-3-phthalimidoazetidine as intermediate compound.

10. Process according to claim 9, characterized in that it consists in hydrolyzing said 1-benzhydryl-3-phthalimidoazetidine and in obtaining the 3-amino-1-benzhydryl azetidine as intermediate compound.

11. Process according to any one of claims 1 to 8, characterized in that it consists in treating the 3-sulfonyloxyazetidine protected in position 1 with an alkali metal phthalimide, in hydrogenating the resulting 3-phthalimidoazetidine, protected in position 1, and in obtaining the 3-phthalimidoazetidine as intermediate compound.

**Patentansprüche** (für die Vertragsstaaten : BE, CH, DE, FR, GB, IT, LI, LU, NL, SE)

1. 3-Aminoazetidin und dessen Additionssalze mit anorganischen oder organischen Säuren.

2. 3-Aminoazetidin-dihydrochlorid.

3. Verfahren zur Herstellung von 3-Aminoazetidin und dessen Salzen, dadurch gekennzeichnet, daß man ein in Position 1 geschütztes 3-Sulfonyloxyazetidin der Formel II

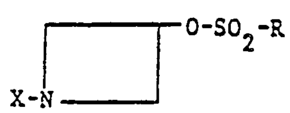

(II)

worin R eine Alkylgruppe mit 1 bis 4 Kohlenstoffatomen, eine Phenylgruppe oder eine Tolylgruppe darstellt und X eine Schutzgruppe, ausgewählt aus den Gruppen Benzyl, Benzhydryl oder Trityl, gegebenenfalls substituiert mit Methoxy und 2,2,2-Trichlorethyl, darstellt, mit einem alkalischen Phthalimid behandelt, das in Position 1 geschützte 3-Phthalimidoazetidin zwei aufeinanderfolgenden Reaktionen, u. zw. einer Hydrolyse mit einer wässerigen 60-90 %igen Hydrazinlösung und einer katalytischen Hydrierung oder, wenn die Schutzgruppe Trityl ist, einer Hydrolyse in leicht sauren Bedingungen, unterzieht und gegebenenfalls das so erhaltene Produkt mit anorganischen oder organischen Säuren in Additionssalze überführt.

4. Verfahren nach Anspruch 3, dadurch gekennzeichnet, daß 1-Benzhydryl-3-mesyloxyazetidin als Ausgangsverbindung verwendet wird.

5. Verfahren nach Anspruch 3, dadurch gekennzeichnet, daß man 1-Benzhydryl-3-mesyloxyazetidin mit einem alkalischen Phthalimid erhitzt, das so erhaltene 1-Benzhydryl-3-phthalimidoazetidin mit einer wässerigen 60-90 %igen Hydrazinlösung hydrolysiert, das so erhaltene 3-Amino-1-benzhydrylazetidin einer katalytischen Hydrierung unterzieht und das so erhaltene 3-Aminoazetidin in seine Additionssalze überführt.

6. Verfahren nach Anspruch 5, dadurch gekennzeichnet, daß 3-Aminoazetidin in Dihydrochloridform isoliert wird.

7. Verfahren nach einem der Ansprüche 3 oder 5, dadurch gekennzeichnet, daß die Reaktion mit dem alkalischen Phthalimid in Gegenwart eines Phasenübergangskatalysators durchgeführt wird.

8. Verfahren nach Anspruch 7, dadurch gekennzeichnet, daß Tetrabutylammonium-bromid oder Hexadecyltributylphosphonium-bromid als Phasenübergangskatalysator verwendet wird.

9. Als neue Zwischenprodukte die Verbindungen der Formel VI

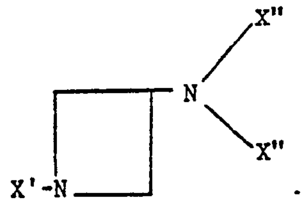

(VI)

worin X' für Wasserstoff oder eine Schutzgruppe, ausgewählt aus den Gruppen Benzyl, Benzhydryl oder Trityl, gegebenenfalls substituiert mit Methoxy und 2,2,2-Trichlorethyl, steht, die beiden X" Wasserstoff oder mit dem Stickstoffatom, an das sie gebunden sind, eine Phthalimidogruppe darstellen, wobei X' und die beiden X" nicht gleichzeitig Wasserstoff sind, und deren Additionssalze mit anorganischen oder organischen Säuren.

10. 1-Benzhydryl-3-phthalimidoazetidin und dessen Additionssalze mit anorganischen oder organischen Säuren.

11. 3-Amino-1-benzhydrylazetidin und dessen Additionssalze mit anorganischen oder organischen Säuren.

12. 3-Amino-1-benzhydrylazetidin-dihydrochlorid.

13. 3-Phthalimidoazetidin und dessen Additionssalze mit anorganischen oder organischen Säuren.

**Patentansprüche** (für den Vertragsstaat AT)

1. Verfahren zur Herstellung von 3-Aminoazetidin und dessen Additionssalzen mit anorganischen oder organischen Säuren, dadurch gekennzeichnet, daß es darin besteht, daß ein in Position 1 geschütztes 3-Sulfonyloxyazetidin der Formel II

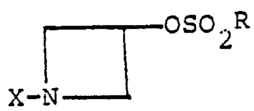

(II)

worin R eine Alkylgruppe mit 1 bis 4 Kohlenstoffatomen, eine Phenylgryppe oder eine Tolylgruppe darstellt und X eine Schutzgruppe, ausgewählt aus den Gruppen Benzyl, Benzhydryl oder Trityl, gegebenenfalls substituiert mit Methoxy und 2,2,2-Trichlorethyl, darstellt, mit einem alkalischen Phthalimid behandelt, das in Position 1 geschützte 3-Phthalimidoazetidin zwei aufeinanderfolgenden Reaktionen, u. zw. einer Hydrolyse mit einer wässerigen 60-90 %igen Hydrazinlösung und einer katalytischen Hydrierung oder, wenn die Schutzgruppe Trityl ist, einer Hydrolyse in leicht sauren Bedingungen, unterzogen wird und gegebenenfalls das so erhaltene Produkt mit anorganischen oder organischen Säuren in Additionssalze übergeführt wird.

2. Verfahren nach Anspruch 1, dadurch gekennzeichnet, daß 3-Aminoazetidin in Dihydrochloridform isoliert wird.

3. Verfahren nach Anspruch 1, dadurch gekennzeichnet, daß 1-Benzhydryl-3-mesyloxyazetidin als Ausgangsverbindung verwendet wird.

4. Verfahren nach Anspruch 1, dadurch gekennzeichnet, daß man 1-Benzhydryl-3-mesyloxyazetidin mit einem alkalischen Phthalimid erhitzt, das so erhaltene 1-Benzhydryl-3-phthalimidoazetidin mit einer wässerigen 60-90 %igen Hydrazinlösung hydrolysiert, das so erhaltene 3-Amino-1-benzhydrylazetidin einer katalytischen Hydrierung unterzieht und das so erhaltene 3-Aminoazetidin in seine Additionssalze überführt.

5. Verfahren nach Anspruch 4, dadurch gekennzeichnet, daß 3-Aminoazetidin in Dihydrochloridform isoliert wird.

6. Verfahren nach einem der Ansprüche 1 oder 4, dadurch gekennzeichnet, daß die Reaktion mit dem alkalischen Phthalimid in Gegenwart eines Phasenübergangskatalysators durchgeführt wird.

7. Verfahren nach Anspruch 6, dadurch gekennzeichnet, daß Tetrabutylammonium-bromid oder Hexadecyltributylphosphonium-bromid als Phasenübergangskatalysator verwendet wird.

8. Verfahren nach einem der Ansprüche 1 bis 7, dadurch gekennzeichnet daß man als Zwischenprodukte Verbindungen der Formel IV

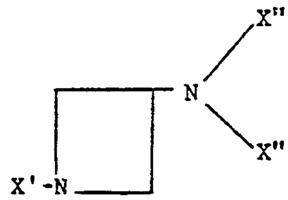

(IV)

worin X' für Wasserstoff oder eine Schutzgruppe, ausgewählt aus den Gruppen Benzyl, Benzhydryl oder Trityl, gegebenenfalls substituiert mit Methoxy und 2,2,2-Trichlorethyl, steht ; die beiden X'' Wasserstoff oder mit dem Stickstoffatom, an das sie gebunden sind, eine Phthalimidogruppe darstellen, wobei X' und die beiden X'' nicht gleichzeitig Wasserstoff sind.

9. Verfahren nach einem der Ansprüche 1 bis 8, dadurch gekennzeichnet, daß es darin besteht, daß in Position 1 mit der Gruppe Benzhydryl geschütztes 3-Sulfonyloxyazetidin mit einem alkalischen Phthalimid behandelt wird und als Zwischenverbindung 1-Benzhydryl-3-phthalimidoazetidin erhalten wird.

10. Verfahren nach Anspruch 9, dadurch gekennzeichnet, daß es darin besteht, daß 1-Benzhydryl-3-phthalimidoazetidin hydrolysiert wird und als Zwischenverbindung 3-Amino-1-benzhydrylazetidin erhalten wird.

11. Verfahren nach einem der Ansprüche 1 bis 8, dadurch gekennzeichnet, daß es darin besteht, daß in Position 1 geschütztes 3-Sulfonyloxyazetidin mit einem alkalischen Phthalimid behandelt wird, das erhaltene, in Position 1 geschützte 3-Phthalimidoazetidin hydriert wird und als Zwischenverbindung 3-Phthalimidoazetidin erhalten wird.